# EUROPEAN PATENT SPECIFICATION

(11) **EP 0 626 938 B1**
(45) Date of publication and mention of the grant of the patent: **14.01.1998**
(21) Application number: 93917425.6
(22) Date of filing: 17.02.1993
(51) Int. Cl.: C07C 41/01, C07C 29/10, C07C 31/20, C07C 41/26, C07C 43/10, C08J 11/14

(54) **PROCESS FOR RECYCLING POLYMERS HAVING REPEATING ETHER LINKAGES**
VERFAHREN ZUM RECYCLING VON POLYMEREN MIT SICH WIEDERHOLENDEN ÄTHERBINDUNGEN
PROCEDE DE RECYCLAGE DE POLYMERES COMPRENANT DES LIAISONS ETHER REPETITIVES

(30) Priority: 20.02.1992 US 838645; 08.01.1993 US 1954
(43) Date of publication of application: 07.12.1994
(73) Proprietor: EXXON RESEARCH AND ENGINEERING COMPANY, Florham Park, New Jersey 07932-0390 (US)
(72) Inventor: SISKIN, Michael, Morristown, NJ 07960 (US); SALEH, Ramzi, Yanni, Flemington, NJ 08822 (US); KNUDSEN, George, Andrew, Scotch Plains, NJ 07076 (US)
(74) Representative: Somers, Harold Arnold
(86) International application number: US9301850
(87) International publication number: WO9316976

(56) References cited:
- US-A- 4 581 470
- US-A- 4 806 658
- US-A- 5 043 486

## Description

The present invention relates to a process for depolymerizing high molecular weight polymers having multiple or repeating ether linkages in the backbone to alcohols, and other monomeric and lower molecular weight oligomeric units.

Generally speaking, waste polymers containing repeating ether linkages are less commercially valuable than the corresponding alcohol and monomeric and lower molecular weight oligomeric products which may be produced by cleavage of (c-o) linkages in the polymer backbone. Such waste polymers are generally not easily biodegradable and due to their high molecular weight also are not water soluble. Current processes, such as described in U.S. Patent 4,806,658, require the addition of catalysts to depolymerize these polymers.

In US-A-5,043,486 there is disclosed a method for cleaving ethers, particularly of the type R(OR')ₙ, having low molecular weights; which in the context of the disclosed ethers means up to 2,000. The ethers are cleaved in water under temperature and autogeneous pressure.

The present invention provides a process for depolymerizing high molecular weight polymers having ether linkages in the backbone, consisting of:
(a) forming, in the absence of a depolymerization catalyst, a mixture of neutral water and a water-insoluble polymer having multiple ether linkages in the backbone and a molecular weight of at least 5,000; and
(b) heating the aqueous mixture to a temperature within the range of from 200°C up to the critical temperature of water under autogeneous pressure of the system for a period of time sufficient to depolymerize said polymer into lower molecular weight compounds including alcohols.

The starting polymers have multiple or repeating ether linkages in the backbone and they are insoluble in water at 25°C. The process of the invention converts these polymers into their corresponding lower molecular weight products, viz. alcohols, monomeric products and lower molecular weight oligomeric products.

The mixture of neutral water and polymer is heated from 200°C to critical water temperature under autogeneous pressure of the system, which temperature is about 374°C. The preferred temperature for the heating is in the range 200°C to 350°C.

The invention is applicable to the cleavage of high molecular weight polymers having multiple or repeating ether linkages in the backbone. Such polymers are typically not water-soluble and will have an average molecular weight of greater than or equal to 5,000, more typically from 20,000 and most typically from 100,000. The polymer preferably has the formula:

R'(OR)ₙOR"

wherein n is a whole number typically equal to or greater than 100, depending on the molecular weight of the polymer and wherein R, R' and R" each is selected from the group consisting of hydrocarbon groups and substituted hydrocarbon groups, wherein the hydrocarbon group and substituted hydrocarbon group may be aliphatic (including linear, branched and cyclic aliphatic), aromatic and mixtures thereof. If aromatic hydrocarbons are present as part of the ether backbone, aromatic hydrocarbons having at least two aromatic rings (e.g., naphthalene) are preferred to be the ether linkage in the polymer chain, rather than only one ring aromatics (e.g., naphthyl-O-phenyl, or naphthyl-O-naphthyl, etc. is preferred over phenyl-O-phenyl) as unsubstituted phenyl-O-phenyl bonds are not degraded easily at reaction conditions, except if one of the phenyl rings is (a) replaced by a higher aromatic ring, (b) replaced by an aliphatic group, or (c) substituted with, e.g., a hydroxyl, amino, alkoxy, etc. group, the ether bond to that ring may be degraded in the process of the present invention. Further, R, R' and R" may be the same or different, provided that R, R' and R" are not all unsubstituted phenyl groups; i.e., selected from the group consisting of aliphatic and higher aromatic hydrocarbon groups and mixtures thereof. Thus, the polymer may be any known polymer having repeating or multiple ether linkages in the backbone, such as linear, block, radial or random polymers (including copolymers). Included in random polymers are those wherein R, R' and R" are distributed randomly throughout the polymer and wherein the R in (OR)ₙ also may be composed of different aliphatic or aromatic hydrocarbon groups/blocks.

The length of the chain of each individual R in the repeating unit, (OR)ₙ, may be any number of carbon atoms, so long as the resulting polymer is essentially water insoluble, preferably having from 1 to 30 carbon atoms, most preferably from 1 to 15 carbon atoms. Additionally, cycloaliphatic and aromatic moieties may be appended to the aliphatics.

The terms "hydrocarbon", "aliphatic" (linear, branched and cyclo-) and "aromatic" as used herein are also intended to be inclusive of such groups containing one or more non-interfering substituent groups replacing hydrogen on the chains or rings of the hydrocarbon units (i.e., substituted hydrocarbon groups). As used herein, the term "non-interfering substituent (group)" means a substituent (group) that is essentially inert; i.e., does not interfere with the process of the present invention, i.e., the course of the depolymerization reaction and such substituent groups may readily be selected by those skilled in the art.

Although ordinary tap water may be used in the process, it is preferred to use distilled or deionized water substantially free of dissolved salts and particularly preferred to use water which has been deoxygenated and is substantially free of dissolved oxygen. Removal of oxygen tends to minimize the occurrence of free radical side reactions during the process.

The amount of water used in forming the mixture with these polymers should be at least equal parts of water and polymer, but preferably at least about a two-fold excess, more preferably about a five-fold excess by weight. In general, the higher the water content of the mixture, the greater the expected conversion and conversion rate of the polymer at any given process temperature within the disclosed temperature range.

Conversely, the higher the process temperature within that range, the less water would be required to give rise to higher conversion rates of the polymer. The preferred water content of the mixture ranges from 1:1 to 10:1 by weight water to weight of polymer with 2:1 to 5:1 parts by weight being the preferred.

The process is conducted by introducing the liquid water and the polymer into a reaction vessel, forming an aqueous mixture thereof and heating the mixture under autogeneous pressure and at a temperature within the range of from 200°C to 374°C, preferably from 200°C to 350°C for a period of time sufficient to depolymerize the polymer by cleaving ether linkages in the polymer backbone. The reaction should be carried out preferably in an inert atmosphere, such as argon or nitrogen, as an aid in excluding oxygen from the system. The polymer may be introduced into the reactor in any form that can suitably be accommodated therein, for example as a powder, small chips or pieces cut to appropriate size.

The term "autogeneous pressure of the system" refers to the combined vapor pressure exerted by the mixed components present in the aqueous system heated at a particular process temperature. The autogeneous pressure of water alone in such a system ranges from 225 psig to 3200 psig over a temperature range of from 200°C up to about 374°C the critical temperature of water. The autogeneous pressure of a system containing both water and the polymer would be higher over this temperature range as a function of the polymer content and the partial pressure exerted by the polymer decomposition products.

The starting materials required in the process of the present invention are liquid water that has a pH of 7 at room temperature, and a polymer containing repeating or multiple ether linkages in the backbone and otherwise having the characteristics described above.

The terms "conversion", "degrading" and "depolymerization" as used herein are defined as cleavage of one or more C-O bonds in the ether linkages of the polymer backbone to produce more desirable, value added, lower molecular weight compounds than the starting polymer (i.e., alcohols, monomeric products and lower molecular weight oligomeric products). Some of these lower molecular weight compounds may be water soluble. Where the starting polymer ranges from 5,000 to over 100,000 in average molecular weight, the product of the reaction may generally comprise a mixture composed primarily of the corresponding alcohol and oligomeric units containing preferably from about 1 to about 15 ether units as the major components. Depending on the length of time that the reaction is carried out, it can effect the cleavage of the resulting oligomers (lower molecular weight compounds), if desired.

The process can be used to convert any weight percent of the starting polymer. Generally the percentage of conversion of polymer which may be achieved in accordance with the process of this invention may range from at least about 75% to quantitative amounts depending upon the number of ether linkages in starting polymer and reaction conditions. Reaction times of 0.1 hour to 8 hours are generally acceptable depending on the amount of water present, the temperature and the amount of conversion desired.

The process of the present invention may be particularly adapted for use in conjunction with other chemical processes wherein polymers with multiple or repeating ether linkages in the backbone are formed as a less valuable byproduct. The advantageous use of the present process in conjunction with other chemical processes where polymers having ether linkages in the backbone are formed as byproducts should be evident to the skilled practitioner.

The process of this invention may be carried out in a batch or in a continuous mode using conventional pressure equipment. Examples of such equipment include a laboratory bomb, a high pressure autoclave, a stirred tank reactor or a continuous flow-through tube, each equipped with a heating means capable of achieving and maintaining the required temperatures and pressures over the required time period.

### EXAMPLES

### Example 1

Polyethylene glycol (1.0 g) (MW 100,000), in a solid, insoluble form, was mixed with H₂O (10.0 g) and heated at 350°C for 2 hours to produce a yellow solution (indicating that depolymerization of the insoluble starting material to lower molecular weight products had occurred).

### Example 2

Polyethylene glycol (1.0 g) (MW 19,000), in a solid, insoluble form, was mixed with H₂O (10.0 g) and heated at 315°C for 1 hour to produce a colorless solution (indicating that depolymerization of the starting material to lower molecular weight products had occurred).

### Example 3

Polyethylene glycol (1.0 g) (MW 100,000), in a solid, insoluble form, was mixed with H₂O (10.0 g) and heated at 315°C to produce a white, cloudy solution. GC mass spec analysis showed alcohol and ether units in range of 1-13 units (indicating that depolymerization of the starting material to lower molecular weight products had occurred).

## Claims

1. A process for depolymerizing high molecular weight polymers having ether linkages in the backbone, consisting of:
(a) forming, in the absence of a depolymerization catalyst, a mixture of neutral water and a water-insoluble polymer having multiple ether linkages in the backbone and a molecular weight of at least 5,000; and
(b) heating the aqueous mixture to a temperature within the range of from 200°C up to the critical temperature of water under autogeneous pressure of the system for a period of time sufficient to depolymerize said polymer into lower molecular weight compounds including alcohols.

2. The process of claim 1, wherein said polymer has the formula:
R'(OR)ₙOR"
wherein the polymer has a molecular weight of at least 5,000 and wherein R, R' and R" are the same or different and each is selected from unsubstituted aliphatic and aromatic hydrocarbon groups and substituted aliphatic and aromatic hydrocarbon groups, wherein at least one of R, R' and R" is not an unsubstituted phenyl group, and wherein n is a whole number of at least 100.

3. The process of claim 1 or claim 2, wherein said aqueous mixture contains from 1:1 to 10:1 parts by weight of water per part by weight of polymer.

4. The process of claim 3, wherein said ratio is from 2:1 to 5:1.

5. The process of any preceding claim, wherein said mixture is heated at a temperature of from 200°C to 350°C.

6. The process of any preceding claim, wherein the polymer is polyethylene glycol.

## Patentansprüche

1. Verfahren zum Depolymerisieren von Polymeren mit hohem Molekulargewicht mit Etherbindungen in dem Grundgerüst, bei dem
(a) in Abwesenheit eines Depolymerisationskatalysators eine Mischung aus neutralem Wasser und wasserunlöslichem Polymer mit mehreren Etherbindungen in dem Grundgerüst und einem Molekulargewicht von mindestens 5 000 gebildet wird, und
(b) die wäßrige Mischung auf eine Temperatur im Bereich von 200°C bis zu der kritischen Temperatur des Wassers unter autogenem Druck des Systems während eines ausreichenden Zeitraums erhitzt wird, um das Polymer zu Verbindungen mit niedrigerem Molekulargewicht einschließlich Alkoholen zu depolymerisieren.

2. Verfahren nach Anspruch 1, bei dem das Polymer die Formel
R'(OR)ₙOR"
hat, wobei das Polymer ein Molekulargewicht von mindestens 5 000 hat und R, R' und R" gleich oder unterschiedlich sind und jeweils ausgewählt sind aus unsubstituierten aliphatischen und aromatischen Kohlenwasserstoffgruppen und substituierten aliphatischen und aromatischen Kohlenwasserstoffgruppen, wobei mindestens einer von R, R' und R" keine unsubstituierte Phenylgruppe ist, und wobei n eine ganze Zahl von mindestens 100 ist.

3. Verfahren nach Anspruch 1 oder Anspruch 2, bei dem die wäßrige Mischung 1:1 bis 10:1 Gewichtsteile Wasser je Gewichtsteil Polymer enthält.

4. Verfahren nach Anspruch 3, bei dem das Verhältnis 2:1 bis 5:1 beträgt.

5. Verfahren nach einem der vorhergehenden Ansprüche, bei dem die Mischung auf eine Temperatur von 200°C bis 350°C erhitzt wird.

6. Verfahren nach einem der vorhergehenden Ansprüche, bei dem das Polymer Polyethylenglykol ist.

## Revendications

1. Procédé permettant de dépolymériser des polymères de poids moléculaire élevé ayant des liaisons éther dans l'ossature, comprenant les étapes suivantes :
(a) on forme, en l'absence d'un catalyseur de dépolymérisation, un mélange d'eau neutre et d'un polymère insoluble dans l'eau ayant des liaisons éther multiples dans l'ossature et un poids moléculaire d'au moins 5000; et
(b) on chauffe le mélange aqueux à une température dans la plage de 200°C à la température critique de l'eau sous pression autogène du système pendant une période de temps suffisante pour dépolymériser ledit polymère en composés de moindres poids moléculaires, notamment des alcools.

2. Procédé selon la revendication 1, dans lequel ledit polymère a pour formule :
R'(OR)ₙOR"
dans laquelle le polymère a un poids moléculaire d'au moins 5000 et dans laquelle, R, R' et R" sont identiques ou différents et chacun d'entre eux est sélectionné parmi des groupes hydrocarbures aliphatiques et aromatiques non substitués et des groupes hydrocarbures aliphatiques et aromatiques substitués, au moins un des groupes R, R' et R" n'étant pas un groupe phényle non substitué et n étant un nombre entier d'au moins 100.

3. Procédé selon la revendication 1 ou 2, dans lequel ledit mélange aqueux contient 1:1 à 10:1 parties en poids d'eau par partie en poids de polymère.

4. Procédé selon la revendication 3, dans lequel ledit rapport est de 2:1 à 5:1.

5. Procédé selon l'une quelconque des revendications précédentes, dans lequel ledit mélange est chauffé à une température de 200 à 350°C.

6. Procédé selon l'une quelconque des revendications précédentes, dans lequel le polymère est un polyéthylèneglycol.
